# EUROPEAN PATENT SPECIFICATION

(11) **EP 2 471 521 B1**
(45) Date of publication and mention of the grant of the patent: **16.08.2017**
(21) Application number: 10836913.3
(22) Date of filing: 12.11.2010
(51) Int. Cl.: A61K 9/22, A61K 31/357, A61P 31/12, A61K 9/20, A61K 9/28, A61K 9/00

(54) **DOUBLE-LAYER OSMOTIC PUMP CONTROLLED RELEASE TABLET OF BICYCLOL AND PREPARATION METHOD THEREOF**
DOPPELSCHICHTIGE, OSMOSEPUMPENGESTEUERTE FREISETZUNGSTABLETTE VON BICYCLOL UND HERSTELLUNGSVERFAHREN DAFÜR
COMPRIMÉ DE BICYCLOL À DEUX COUCHES À LIBÉRATION CONTRÔLÉE INTÉGRANT UNE POMPE OSMOTIQUE ET PROCÉDÉ DE PRÉPARATION ASSOCIÉ

(30) Priority: 15.12.2009 CN 200910242393
(43) Date of publication of application: 04.07.2012
(73) Proprietor: Beijing Union Pharmaceutical Factory, Beijing 102600 (CN)
(72) Inventor: GUO, Mintong, Beijing 100176 (CN); LV, Zhaoyun, Beijing 102600 (CN); LI, Yan, Beijing 100050 (CN); PAN, Xiandao, Beijing 102600 (CN); YANG, Chaolian, Beijing 100176 (CN); LI, Qiang, Beijing 102600 (CN); WANG, Xiaomei, Beijing 102600 (CN); LAN, Pei, Beijing 102600 (CN); ZHAO, Limin, Beijing 102600 (CN); LIU, Yujuan, Beijing 100176 (CN); LIU, Yan, Beijing 100176 (CN)
(74) Representative: Witte, Weller & Partner Patentanwälte mbB
(86) International application number: PCT/CN2010/001817
(87) International publication number: WO 2011/072474

(56) References cited:
- CN-A- 1 439 372
- CN-A- 1 608 621
- CN-A- 1 628 640
- CN-A- 1 846 668
- US-A- 6 146 662
- US-A1- 2005 058 707
- GENG LIU: "Bicyclol: A Novel Drug for Treating Chronic Viral Hepatitis B and C", MEDICINAL CHEMISTRY, vol. 5, no. 1, January 2009 (2009-01), pages 29-43, XP055064166, ISSN: 1573-4064, DOI: 10.2174/157340609787049316

## Description

### FIELD OF THE INVENTION

The present invention relates to a controlled release dosage form of bicyclol for treating hepatitis, in particular, relates to a double-layer osmotic pump controlled release dosage form and preparation method thereof.

### BACKGROUND OF THE INVENTION

An osmotic pump tablet is a new type of controlled release tablets. It is prepared by compressing a drug and a penetration-promoting agent with high osmotic pressure or other excipient(s) into a solid tablet core, coating a controlled release semi-permeable film on the surface thereof and then drilling one or more drug-release orifices on the coating film by laser. After administration, the water in the gastrointestinal tract entered the tablet core through the semi-permeable film, so that the drug is dissolved or suspended to obtain a saturate solution or suspension, further, the excipients with high osmotic pressure are dissolved, providing a high osmotic pressure solution within the film of the tablet. The great difference of osmotic pressure between the inside and the outside of the film drives water enter in the film constantly, and the drug solution or suspension is pumped out through the drug-release orifices constantly.

At present, osmotic pump controlled-release tablets are mainly present in two forms in clinical applications. One form is a multi-compartment osmotic pump controlled-release tablet, and the other form is a mono-compartment elementary osmotic pump controlled-release tablet. Mono-compartment osmotic pump controlled-release tablets are more commonly used for water-soluble drugs. The tablet core contains only one layer of drug and a penetration-promoting agent with high osmotic pressure. The coating film is formed by a polymer which is coated on the surface of the tablet core,
and one or two drug-release orifices exist on the coating film. Multi-compartment osmotic pump controlled-release tablets are suitable for preparation of water-soluble or insoluble drugs. The tablet core thereof is usually a double-layer tablet, and the drug layer consists of a drug, a penetration-promoting agent and other excipients. Upon contacting with water, the drug solution or drug suspension is formed. The push layer consists of an inorganic salt and a swellable polymer. When water enters into the push layer of the tablet core, the polymer swells after absorbing water to promote the drug solution or suspension of the drug layer discharged from the tablet. Compared with mono-compartment osmotic pump controlled-release tablets, the drug release of double-compartment osmotic pump controlled-release tablets is more close to the zero-order release. However, fewer products are currently marketed due to the difficulties of the preparing process and design of double-compartment controlled-release tablets.

Oral osmotic pump formulations can avoid the considerable fluctuation of blood concentration, reduce administration times and toxic or side effects of the drug, and improve patients' compliance and safety compared with common oral formulations. Compared to sustained-release formulations, the drug release of oral osmotic pump controlled release formulations is more stable, and closer to zero-order release, and the release behavior is not influenced by the gastrointestinal tract variables (for example, the frequency of gastrointestinal peristalsis, pH and gastrointestinal emptying time), thereby reducing individual differences of the drug.

Bicyclol is an innovative drug with independent intellectual property rights developed by Institute of Materia Medica, Chinese Academy of Medical Sciences, used for the treatment of elevated aminotransferase caused by chronic hepatitis. The half-life of bicyclol is short, so it is required to administer bicyclol three times per day in clinical treatments, which is extremely inconvenient for chronic hepatitis B patients who require long-term administration. In addition, bicyclol is released fast after oral administration, and individual differences are great, thereby the therapeutic effects are compromised.

CN 1846668 A discloses a pharmaceutical composition osmotic pump controlled release formulation. One of the active ingredients proposed is bicyclol. A sustained release formulation is disclosed comprising a tablet core including inter alia the medicament and the excipient polyvinylpyrrolidone. The tablet core has a coating with pores formed therein.

CN 1608621 A discloses a delayed release preparation of bicyclol. The preparation includes HPMC as one of the preferred hydrophilic gels used for the skeleton material. Preferred retardants employed comprise aliphatic acid or aliphatic alcohol.

US 6,146,662 A discloses a dosage form for delayed-drug delivery. The drug is delivered during a drug-delivery period at a controlled rate over time. A proposed dosage form comprises inter alia verapamil hydrochloride, poly(ethylene oxide) having a molecular weight of 300,000, and polyvinylpyrrolidone having a molecular weight of 38,000.

US 2005/058707 A1 discloses compositions and dosage forms for enhanced dispersion of dopiramate in a controlled release dosage form delivered as a dry or substantially dry erodible solid at a uniform rate over a prolonged period of time.

In the present invention, bicyclol is formulated into osmotic pump tablet, and bicyclol can be released at a slow and constant rate and the requirements of zero-order release are achieved, thus, the peak trough ratio of plasma drug concentration could be reduced, and the adverse effects of common tablets could be reduced. In addition, the drug release behavior of bicyclol osmotic pump controlled-release tablet is not influenced by the factors such as environmental pH, gastrointestinal motility and food, so individual differences are reduced. The solubility of bicyclol is low and thus affects the absorption and utilization of bicyclol. The slow and constant release characteristics of bicyclol osmotic pump tablets can significantly improve the absorption of bicyclol, thus improve the clinical efficacy of bicyclol.

### SUMMARY OF THE INVENTION

One object of the present invention is to provide an osmotic pump controlled release tablet of bicyclol.

Another object of the present invention is to provide a method for preparing a double-layer osmotic pump controlled release tablet of bicyclol.

The present invention provides a double-layer osmotic pump controlled release tablet of bicyclol using osmotic pressure as the main release force and preparation method thereof. The present invention is as defined in the appended claims.

The technical solution of the invention is achieved by the following steps: double-layer osmotic pump controlled-release tablets are prepared using bicyclol as active ingredient, and the tablet core contains two layers, wherein one layer is a drug layer which is formed by mixing the active drug bicyclol and other excipients such as a polymer and/or a binder; another layer is a push layer which consists of other excipients such as a polymer, a pigment, a binder and the like. For a double-layer osmotic pump controlled release tablet, the drug layer and the push layer are compressed into the tablet core, then a semi-permeable film is coated on the double-layer tablet prepared, finally, one or more drug-release orifices are drilled on the drug layer by laser or other suitable means.

The weight percentage of each component of the bicyclol double-layer osmotic pump controlled-release tablet according to the present invention is as follows:

| | |
|---|---|
| bicyclol | 1-40% |
| excipients for controlled release in the drug layer of the tablet core | 20-70% |
| excipients for controlled release in the push layer of the tablet core | 20-70% |
| other excipients | as balance |

The tablet core of the controlled-release tablet according to the present invention includes a drug layer and a push layer, wherein the excipient for controlled release in the drug layer is a polymer. According to the requirements for drug release, one or more inorganic salts or other excipients or a mixture thereof can be added thereto. The polymer is preferably a polyoxyethylene polymer, hydroxypropyl methylcellulose and povidone. Experiments have showed that the quality of the preparaton is better when the molecular weight of the polyoxyethylene polymer is 100,000 to 300,000 and the molecular weight of povidone is 30,000-70,000. The inorganic salt is preferably sodium chloride.

The excipient for controlled release in the push layer is one or more selected from the group consisting of a polyoxyethylene polymer, hydroxypropyl methylcellulose, sodium chloride and povidone, or a mixture thereof. Experiments have showed that suitable molecular weight of polyoxyethylene polymer is 5,000,000 to 7,500,000.

In order to improve the compressibility of the raw materials and improve the appearance of tablets, thereby the appropriate performance could be achieved, the tablet core may comprise, in addition to the main excipients, other excipients known in the pharmaceutical art including one or more selected from the group consisting of binders, lubricants, glidants, coloring agents, fillers, suspending agents, stabilizers, solubilizers, absorption enhancers, surfactants and solvents.

The controlled release coating film for bicyclol osmotic pump controlled release tablets is prepared by spraying the coating solution onto the double-layer tablet core and then drying. The coating solution is composed mainly of a film-forming material and a solvent. A plasticizer or a pore-forming agent can be added as needed.

Suitable coating materials include either one of cellulose acetate and ethyl cellulose, or a mixture thereof.

The plasticizers include one or more selected from the group consisting of methyl phthalate, ethyl phthalate, triethyl citrate, polyethylene and glycol, and a mixture thereof. More preferably, the molecular weight of the polyethylene glycol is 200 to 8000, even more preferably, the molecular weight of the polyethylene glycol is 400 to 4000.

The pore-forming agents include one or more selected from the group consisting of glycerin, propylene glycol, polyethylene glycol and sugars, and a mixture thereof, wherein sugars may include a monosaccharide, a disaccharide and a mixture thereof known in the pharmaceutical art such as sucrose and glucose.

The coating solution is prepared using an appropriate solvent, and the solvent can be one or more selected from the group consisting of acetone, water, ethanol, methylene chloride, methanol and isopropyl alcohol, and a mixture thereof.

In order to obtain an appropriate coating color, or to improve the quality index such as adhesive property, other excipients known in the pharmaceutical art may be added to the coating solution, including one or more selected from the group consisting of binders, lubricants, coloring agents, opacifying agents and surfactants as needed.

For the bicyclol osmotic pump controlled release tablets obtained according to the present invention, the drug mainly releases from the drug-release orifices on the insoluble coating film. The drug-release orifices are present on the surface of the drug layer. One or more drug-release orifices can exist according to the need of release rate. The drug-release orifices can be obtained by laser drilling or other mechanical drilling methods, and the laser drilling method is a mature and applicable method that can achieve industrial continuous production.

The preparation method of the bicyclol double-layer osmotic pump controlled release tablets according to the present invention can be prepared generally by the following steps:
A. preparing the double-layer tablets; B. coating the controlled release film, and C. drilling the controlled release film, wherein the double-layer tablets can be prepared by compression after granulation or direct powder compression. Experiments show that both of the two methods can obtain controlled-release tablets with good appearance and qualified quality.

For the purpose of good appearance and moisture-proofing, a moisture-proofing film can be coated after the controlled release film is drilled, so as to ultimately obtain commercial products with good appearance and qualified quality.

Bicyclol is prepared into osmotic pump tablets in the invention. When a bicyclol controlled-release tablet enters into the body, the controlled-release tablet absorbs water in the gastrointestinal tract. The polymer in the drug layer becomes fluid-like form by absorbing water and forms a suspension with the active drug, at the same time, the volume of the polymer in the push layer increases constantly after absorbing water, thereby pushing the drug from the drug layer release uniformly from the drug-release orifices. The active ingredients of bicyclol controlled-release tablets can be released in a slow and constant manner to achieve the zero-order release. The drug release behavior is not influenced by the factors such as environmental pH, gastrointestinal motility and food, so there is a good correlation between in vivo and in vitro release. Bicyclol osmotic pump tablets can reduce the differences between peaks and valleys of drug plasma concentration, reduce the adverse effects of ordinary tablets, enhance the absorption of insoluble bicyclol, reduce individual differences, and thereby improve the clinical efficacy of bicyclol.

### DETAILED DESCRIPTION OF THE INVENTION

To further illustrate the innovation embodied in the bicyclol double-layer osmotic pump controlled release tablets and the preparation method thereof according to the present invention, the present invention is further described in combination with the following examples, but the protection scope of the present invention is not merely limited to the contents described in the examples.

### Reference Example 1:

**drug layer**

| | |
|---|---|
| bicyclol | 75mg |
| poly(ethylene oxide) | 134mg |
| PVP K30 | 24mg |
| sodium chloride | 5mg |
| magnesium stearate | 2mg |
| total weight of the drug layer | 240mg |

**push layer**

| | |
|---|---|
| poly(ethylene oxide) | 64mg |
| PVP K30 | 10mg |
| sodium chloride | 35mg |
| iron oxide red | 0.6mg |
| magnesium stearate | 0.4mg |
| total weight of the push layer | 110mg |

**semi-permeable coating**

| | |
|---|---|
| cellulose acetate | 3.9g |
| PEG 4000 | 0.2g |
| 95% acetone/water | 1000ml |

### Preparation method

1) Preparation of the drug layer: a prescribed amount of bicyclol was mixed with the excipients except magnesium stearate. The resulting mixture was granulated by adding an appropriate amount of ethanol and dried at room temperature or in a dryer.
2) The dried drug layer was sieved, then thoroughly mixed with a prescribed amount of magnesium stearate.
3) Preparation of the push layer: a prescribed amount of poly(ethylene oxide) was mixed with the excipients except magnesium stearate. The resulting mixture was granulated by adding an appropriate amount of ethanol and dried at room temperature or in a dryer.
4) The dried push layer was sieved, then thoroughly mixed with a prescribed amount of magnesium stearate.
5) The drug layer and the push layer prepared were compressed into tablets using a double layer tablet press machine according to the tablet weight.
6) The double-layer tablets prepared were coated in a coating machine to achieve an appropriate weight gain.
7) The coated double-layer tablets were dried to remove the residue solvent.
8) Drilling: the coated tablets were drilled on the surface of the drug layer by laser drilling.
9) Coating the isolating coating: a layer of brown-red moisture-proof film coating was coated on the surface of coated double-layer tablets prepared.

### Example 2:

**drug layer**

| | |
|---|---|
| bicyclol | 75mg |
| poly(ethylene oxide) | 139mg |
| hydroxypropyl methyl cellulose | 24mg |
| magnesium stearate | 2mg |
| total weight of the drug layer | 240mg |

**push layer**

| | |
|---|---|
| poly(ethylene oxide) | 64mg |
| hydroxypropyl methyl cellulose | 10mg |
| sodium chloride | 35mg |
| iron oxide red | 0.6mg |
| magnesium stearate | 0.4mg |
| total weight of the push layer | 110mg |

**semi-permeable coating**

| | |
|---|---|
| cellulose acetate | 95g |
| PEG 3350 | 5g |
| dichloromethane/methanol (2:10) | 2000ml |

### Preparation method

1) Preparation of the drug layer: a prescribed amount of bicyclol was mixed with the excipients except magnesium stearate. The resulting mixture was granulated by adding an appropriate amount of ethanol and dried at room temperature or in a dryer.
2) The dried drug layer was sieved, then thoroughly mixed with a prescribed amount of magnesium stearate.
3) Preparation of the push layer: a prescribed amount of poly(ethylene oxide) was mixed with the excipients except magnesium stearate. The resulting mixture was granulated by adding an appropriate amount of ethanol and dried at room temperature or in a dryer.
4) The dried push layer was sieved, then thoroughly mixed with a prescribed amount of magnesium stearate.
5) The drug layer and the push layer prepared were compressed into tablets using a double layer tablet press machine according to the tablet weight.
6) The double-layer tablets prepared were coated in a coating machine to achieve an appropriate weight gain.
7) The coated double-layer tablets were dried to remove the residue solvent.
8) Drilling: the coated tablets were drilled on the surface of the drug layer by laser drilling.
9) Coating the isolating coating: a layer of brown-red moisture-proof film coating was coated on the surface of coated double-layer tablets prepared.

### Example 3

**drug layer**

| | |
|---|---|
| bicyclol | 165mg |
| poly(ethylene oxide) | 393mg |
| HPMC | 70mg |
| magnesium stearate | 7mg |
| total weight of the drug layer | 635mg |

**push layer**

| | |
|---|---|
| poly(ethylene oxide) | 140mg |
| hydroxypropyl methyl cellulose | 20mg |
| sodium chloride | 71mg |
| iron oxide red | 1.5mg |
| magnesium stearate | 2.5mg |
| total weight of the push layer | 235mg |

**semi-permeable coating**

| | |
|---|---|
| cellulose acetate | 95g |
| PEG | 5g |
| dichloromethane/methanol (2:10) | 2000ml |

### Preparation method

Similar to Example 2, the drug layer and the push layer were firstly prepared, and then compressed into double-layer tablets, subsequently, a semi-permeable film was coated, finally, the laser drilling and a moisture-proof isolating coating was performed.

### Reference Example 4

**drug layer**

| | |
|---|---|
| bicyclol | 25mg |
| poly(ethylene oxide) | 45mg |
| PVP K30 | 8mg |
| sodium chloride | 1mg |
| magnesium stearate | 1mg |
| total weight of the drug layer | 80mg |

**push layer**

| | |
|---|---|
| poly(ethylene oxide) | 30mg |
| PVP K30 | 3mg |
| sodium chloride | 12mg |
| iron oxide red | 0.2mg |
| magnesium stearate | 0.1 mg |
| total weight of the push layer | 45.3mg |

**semi-permeable coating**

| | |
|---|---|
| cellulose acetate | 3.9g |
| PEG | 0.2g |
| 95% acetone/water | 1000ml |

### Preparation method

Similar to Example 2, the drug layer and the push layer were firstly prepared, and then compressed into double-layer tablets, subsequently, a semi-permeable film was coated, finally, the laser drilling and a moisture-proof isolating coating was performed.

### Example 5

**drug layer**

| | |
|---|---|
| bicyclol | 75mg |
| poly(ethylene oxide) | 144mg |
| HPMC | 19mg |
| magnesium stearate | 2mg |
| total weight of the drug layer | 240mg |

**push layer**

| | |
|---|---|
| poly(ethylene oxide) | 68mg |
| HPMC | 10mg |
| sodium chloride | 31mg |
| iron oxide red | 0.6mg |
| magnesium stearate | 0.4mg |
| total weight of the push layer | 110mg |

**semi-permeable coating**

| | |
|---|---|
| cellulose acetate | 3.9g |
| PEG 4000 | 0.2g |
| 95% acetone/water | 1000ml |

### Preparation method

1) Preparation of the drug layer: a prescribed amount of bicyclol was mixed with the excipients except magnesium stearate, and then mixed with a prescribed amount of magnesium stearate.
2) Preparation of the push layer: a prescribed amount of poly(ethylene oxide) was mixed with the excipients except magnesium stearate, and then mixed with a prescribed amount of magnesium stearate.
3) The drug layer and the push layer prepared were compressed into tablets using a double layer tablet press machine according to the tablet weight.
4) The double-layer tablets prepared were coated in a coating machine to achieve an appropriate weight gain.
5) The coated double-layer tablets were dried to remove the residue solvent.
6) Drilling: the coated tablets were drilled on the surface of the drug layer by laser drilling.
7) Coating the isolating coating: a layer of brown-red moisture-proof film coating was coated on the surface of coated double-layer tablets prepared.

### Example 6: pharmacokinetic experiment in animals

In order to verify the controlled-release properties of bicyclol osmotic pump controlled-release tablets in vivo, single and multiple oral pharmacokinetic studies on the bicyclol osmotic pump controlled-release tablets prepared in Example 2 was performed in Beagle dogs. The pharmacokinetic studies in Beagle dogs demonstrated that the controlled-release tablets according to the present invention have a long-term controlled-release property.

Experimental animals: Beagle dogs (3 male and 3 female) weighing 5.8-6.6kg were used.

Test method: The Beagle dogs were randomly divided into 2 groups (3 dogs per group). Cross-dose administration was used. There was a wash-out period of one week between the two oral administration, and the background plasma was determined before administration. The dose of bicyclol for ordinary tablet was 75mg per dog once, and the dose for the controlled-release tablet was 75mg per dog once. After administration, blood were sampled at different time points and measured respectively, and the pharmacokinetic parameters were calculated according to the plasma concentration data.

**Table 1: The plasma pharmacokinetic parameters after single oral administration of bicyclol ordinary tablets and controlled-release tablets in Beagle dogs**

| **parameters of statistical moment** | **unit** | **controlled-release tablet** | **general tablet** |
|---|---|---|---|
| **AUC(0-t)** | **mg/L*h** | **1.19 ±0.59** | **1.40±0.27** |
| **AUC(0-∞)** | **mg/L*h** | **2.30 ±1.67** | **1.52±0.32** |
| **NIRT(0-t)** | **h** | **9.09 ±3.61** | **5.54±1.76** |
| **MRT(0-∞)** | **h** | **26.81 ±28.24** | **7.74±2.68** |
| **t1/2z** | **h** | **16.67 ±21.62** | **5.91±3.90** |
| **Tmax** | **h** | **6.17 ±4.58** | **0.31±0.13** |
| **Cmax** | **ug/L** | **157.86 ±45.13** | **703.21±177.10** |

### BRIEF DESCRIPTION OF THE DRAWINGS

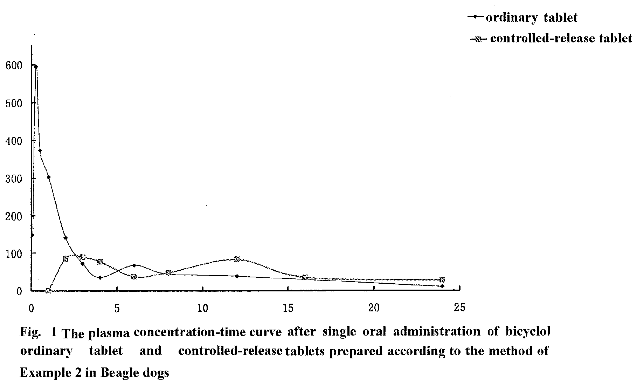

Figure 1: The plasma concentration-time curve of single oral administration of bicyclol ordinary tablets and controlled-release tablets prepared according to the method of Example 2 in Beagle dogs.

## Claims

1. A bicyclol double-layer osmotic pump controlled release tablet, **characterized in that** the tablet comprises bicyclol and pharmaceutically acceptable excipients, and it comprises structurally a double-layer tablet core consisting of a drug layer and a push layer, wherein the tablet core is coated with a water-insoluble semipermeable film, and the semipermeable film has one or several drug-release orifices thereon, wherein the excipients used in the drug layer of the double-layer tablet core comprise polyoxyethylene polymer and hydroxypropyl methylcellulose; and the excipients used in the push layer of the double-layer tablet core comprise polyoxyethylene polymer and hydroxypropyl methylcellulose.

2. The bicyclol osmotic pump controlled release tablet according to claim 1, **characterized in that** the amount of bicyclol is 0.1 mg to 250mg.

3. The bicyclol osmotic pump controlled release tablet according to claim 2, **characterized in that** the amount of bicyclol is 25 mg to 250 mg.

4. The bicyclol osmotic pump controlled release tablet according to claim 2, **characterized in that** the amount of bicyclol is 50 mg to 150 mg.

5. The bicyclol osmotic pump controlled release tablet according to any one of claims 1 to 4, **characterized in that** the tablet core further comprises other excipients known in the pharmaceutical art including one or more selected from the group consisting of binders, lubricants, glidants, coloring agents, fillers, suspending agents, stabilizers, solubilizers, absorption enhancers, surfactants and solvents.

6. The bicyclol osmotic pump controlled release tablet according to claim 1, **characterized in that** the insoluble controlled release coating film is coated on the tablet core, and the controlled release coating film is formed by spraying the coating solution onto the double-layer tablet core and then drying, wherein the coating solution is composed of a film-forming material and a solvent, together with a plasticizer and/or a pore-forming agent; the film-forming material includes either one of cellulose acetate and ethyl cellulose and a mixture thereof; the plasticizers include one or more selected from the group consisting of methyl phthalate, ethyl phthalate, triethyl citrate and polyethylene glycol, and a mixture thereof; the pore-forming agents include one or more selected from the group consisting of glycerin, propylene glycol, polyethylene glycol and sugars, and a mixture thereof, wherein suitable sugars include a monosaccharide, a disaccharide and a mixture thereof known in the pharmaceutical art such as sucrose and glucose; the solvent includes a one or more selected from the group consisting of acetone, water, ethanol, methylene chloride, methanol and isopropyl alcohol, and a mixture thereof.

7. The bicyclol osmotic pump controlled release table according to claim 6, wherein the plasticisers comprise polyethylene glycol and the molecular weight of the polyethylene glycol is 200 to 8000.

8. The bicyclol osmotic pump controlled release table according to claim 6, wherein the plasticisers comprise polyethylene glycol and the molecular weight of the polyethylene glycol is 400 to 4000.

9. The bicyclol osmotic pump controlled release tablet according to any one of claims 1 or 6 to 8, wherein other excipients known in the pharmaceutical art may be added as needed to the coating solution, including one or more selected from the group consisting of binders, lubricants, coloring agents, opacifying agents and surfactants.

10. The bicyclol osmotic pump controlled release tablet according to claim 1, **characterized in that** there is one or more drug-release orifices on the insoluble coating film, and the drug-release orifices open at the surface of the drug layer, wherein the drug-release orifices are obtained by laser drilling or other mechanical drilling methods.

11. The bicyclol osmotic pump controlled release tablet according to claim 10, wherein the drug-release orifices are obtained by laser drilling method.

12. A method for preparing the bicyclol osmotic pump controlled release tablet according to claim 1, **characterized in that** it comprises the following three steps: (1) preparing the double-layer tablets, (2) coating the controlled release film, and (3) drilling the controlled release film, wherein the double-layer tablets are prepared by compression after granulation or direct powder compression.

13. The method according to claim 12, further comprising coating a moisture-proofing coating after the controlled release film is drilled.

## Patentansprüche

1. Bicyclol-Zweischicht-Osmosepumpe-Retardtablette, **dadurch gekennzeichnet, dass** die Tablette Bicyclol und pharmazeutisch verträgliche Trägerstoffe aufweist, und sie strukturell einen Zweischicht-Tablettenkern aufweist, der aus einer Arzneimittelschicht und einer Ausstoßschicht besteht, wobei der Tablettenkern mit einem wasserunlöslichen semipermeablen Film beschichtet ist, und der semipermeable Film eine oder mehrere Öffnungen zur Arzneimittelfreisetzung darauf aufweist, wobei die in der Arzneimittelschicht des Zweischicht-Tablettenkerns verwendeten Trägerstoffe Polyoxyethylenpolymer und Hydroxypropylmethylcellulose aufweisen; und die in der Ausstoßschicht des Zweischicht-Tablettenkerns verwendeten Trägerstoffe Polyoxyethylenpolymer und Hydroxypropylmethylcellulose aufweisen.

2. Bicyclol-Osmosepumpe-Retardtablette gemäß Anspruch 1, **dadurch gekennzeichnet, dass** die Menge an Bicyclol von 0,1mg bis 250 mg beträgt.

3. Bicyclol-Osmosepumpe-Retardtablette gemäß Anspruch 2, **dadurch gekennzeichnet, dass** die Menge an Bicyclol von 25 mg bis 250 mg beträgt.

4. Bicyclol-Osmosepumpe-Retardtablette gemäß Anspruch 2, **dadurch gekennzeichnet, dass** die Menge an Bicyclol von 50 mg bis 150 mg beträgt.

5. Bicyclol-Osmosepumpe-Retardtablette gemäß einem der Ansprüche 1 bis 4, **dadurch gekennzeichnet, dass** der Tablettenkern ferner andere in der pharmazeutischen Fertigkeit bekannte Trägerstoffe einschließlich eines oder mehreren ausgewählt unter der Gruppe bestehend aus Bindemitteln, Gleitmitteln, Fließregulierungsmitteln, Farbstoffen, Füllstoffen, Absetzverhinderungsmitteln, Stabilisatoren, Lösungsvermittlern, Absorptionspromotoren, Tensiden und Lösungsmitteln aufweist.

6. Bicyclol-Osmosepumpe-Retardtablette gemäß Anspruch 1, **dadurch gekennzeichnet, dass** der unlösliche Beschichtungsfilm zur kontrollierten Freisetzung auf den Tablettenkern beschichtet ist, und der Beschichtungsfilm zur kontrollierten Freisetzung durch Sprühen der Beschichtungslösung auf den Zweischicht-Tablettenkern und anschließendes Trocknen gebildet ist, wobei die Beschichtungslösung aus einem filmbildenden Material und einem Lösungsmittel, zusammen mit einem Weichmacher und/oder einem porenbildenden Agens, zusammengesetzt ist; wobei das filmbildende Material entweder eines von Celluloseacetat und Ethylcellulose und eine Mischung davon aufweist; wobei die Weichmacher eines oder mehrere ausgewählt unter der Gruppe bestehend aus Methylphthalat, Ethylphthalat, Triethylcitrat und Polyethylenglycol, und eine Mischung davon aufweisen; wobei die porenbildenden Mittel eines oder mehrere ausgewählt unter der Gruppe bestehend aus Glycerin, Propylenglycol, Polyethylenglycol und Zuckern, und eine Mischung davon aufweisen, wobei geeignete Zucker ein Monosaccharid, ein Disaccharid und eine Mischung davon aufweisen, die in der pharmazeutischen Fertigkeit bekannt sind, wie bspw. Sucrose und Glucose; wobei das Lösungsmittel eines oder mehrere ausgewählt unter der Gruppe bestehend aus Aceton, Wasser, Ethanol, Methylenchlorid, Methanol und Isopropylalcohol, und eine Mischung davon aufweist.

7. Bicyclol-Osmosepumpe Retardtablette gemäß Anspruch 6, wobei die Weichmacher Polyethylenglycol aufweisen und das Molekulargewicht des Polyethylenglycols von 200 bis 8000 beträgt.

8. Bicyclol-Osmosepumpe Retardtablette gemäß Anspruch 6, wobei die Weichmacher Polyethylenglycol und das Molekulargewicht des Polyethylenglycols von 400 bis 4000 beträgt.

9. Bicyclol-Osmosepumpe-Retardtablette gemäß einem der Ansprüche 1 oder 6 bis 8, wobei andere in der pharmazeutischen Fertigkeit bekannte Trägerstoffe zu der Beschichtungslösung nach Bedarf hinzugefügt werden können, einschließlich eines oder mehrere ausgewählt unter der Gruppe bestehend aus Bindemitteln, Gleitmitteln, Farbstoffen, Trübungsmitteln und Tensiden.

10. Bicyclol-Osmosepumpe-Retardtablette gemäß Anspruch 1, **dadurch gekennzeichnet, dass** eine oder mehrere Öffnungen zur Arzneimittelfreisetzung auf dem unlöslichen Beschichtungsfilm vorliegen, und die Öffnungen zur Arzneimittelfreisetzung an der Oberfläche der Arzneimittelschicht offen sind, wobei die Öffnungen zur Arzneimittelfreisetzung durch Laserbohren oder andere mechanische Bohrverfahren erhalten sind.

11. Bicyclol-Osmosepumpe-Retardtablette gemäß Anspruch 10, wobei die Öffnungen zur Arzneimittelfreisetzung durch ein Laserbohrverfahren erhalten sind.

12. Verfahren zur Herstellung der Bicyclol-Osmosepumpe-Retardtablette gemäß Anspruch 1, **dadurch gekennzeichnet, dass** es die nachstehend aufgeführten drei Schritte aufweist: (1) Herstellen der Zweischicht-Tabletten, (2) Beschichten mit dem Film zur kontrollierten Freisetzung, und (3) Bohren des Films zur kontrollierten Freisetzung, wobei die Zweischicht-Tabletten durch Verdichtung nach Granulation oder unmittelbare Pulververdichtung hergestellt sind.

13. Verfahren gemäß Anspruch 12, ferner aufweisend Beschichten mit einer Feuchtigkeitsschutzschicht, nachdem der Film zur kontrollierten Freisetzung gebohrt ist.

## Revendications

1. Comprimé à libération contrôlée de type pompe osmotique à double couche de bicyclol, **caractérisé en ce que** le comprimé comprend le bicyclol et des excipients pharmaceutiquement acceptables, et qu'il comprend structurellement un noyau de comprimé à double couche constitué d'une couche de médicament et d'une couche de répulsion, où le noyau de comprimé est enrobé d'une pellicule semi-perméable insoluble dans l'eau, et la pellicule semi-perméable présente à sa surface un ou plusieurs orifices de libération de médicament, où les excipients utilisés dans la couche de médicament du noyau de comprimé à double couche comprennent un polymère de polyoxyéthylène et de l'hydroxypropylméthylcellulose ; et les excipients utilisés dans la couche de répulsion du noyau de comprimé à double couche comprennent le polymère de polyoxyéthylène et l'hydroxypropylméthylcellulose.

2. Comprimé à libération contrôlée de type pompe osmotique à double couche de bicyclol selon la revendication 1, **caractérisé en ce que** la quantité de bicyclol est comprise entre 0,1 et 250 mg.

3. Comprimé à libération contrôlée de type pompe osmotique à double couche de bicyclol selon la revendication 2, **caractérisé en ce que** la quantité de bicyclol est comprise entre 25 et 250 mg.

4. Comprimé à libération contrôlée de type pompe osmotique à double couche de bicyclol selon la revendication 2, **caractérisé en ce que** la quantité de bicyclol est comprise entre 50 et 150 mg.

5. Comprimé à libération contrôlée de type pompe osmotique à double couche de bicyclol selon l'une quelconque des revendications 1 à 4, **caractérisé en ce que** le noyau de comprimé comprend en outre d'autres excipients connus de l'homme du métier pharmaceutique incluant un ou plusieurs éléments choisis dans le groupe constitué par les suivants : agents liants, lubrifiants, agents glissants, agents colorants, charges, agents de suspension, stabilisants, solubilisants, amplificateurs d'absorption, tensioactifs et solvants.

6. Comprimé à libération contrôlée de type pompe osmotique à double couche de bicyclol selon la revendication 1, **caractérisé en ce que** la pellicule d'enrobage à libération contrôlée insoluble enrobe le noyau de comprimé, et **en ce que** la pellicule d'enrobage à libération contrôlée est formée par la pulvérisation de la solution d'enrobage sur le noyau de comprimé à double couche puis son séchage, où la solution d'enrobage est composée d'une matière filmogène et d'un solvant, ainsi que d'un plastifiant et/ou d'un agent de formation de pores ; la matière filmogène inclut soit de l'acétate de cellulose, soit de l'éthylcellulose, soit un mélange de ceux-ci ; les plastifiants incluent un ou plusieurs éléments choisis dans le groupe constitué par les suivants : phtalate de méthyle, phtalate d'éthyle, citrate de triéthyle et polyéthylène glycol, et un mélange de ceux-ci ; les agents de formation de pores incluent un ou plusieurs éléments choisis dans le groupe constitué par les suivants : glycérine, propylène glycol, polyéthylène glycol et sucres, et un mélange de ceux-ci, où les sucres adaptés incluent un monosaccharide, un disaccharide et un mélange de ceux-ci connu de l'homme du métier pharmaceutique tel que sucrose et glucose ; le solvant inclut un ou plusieurs éléments choisis dans le groupe constitué par les suivants : acétone, eau, éthanol, dichlorométhane, méthanol et isopropanol, ainsi que leurs mélanges.

7. Comprimé à libération contrôlée de type pompe osmotique à double couche de bicyclol selon la revendication 6, où les plastifiants comprennent le polyéthylène glycol et la masse moléculaire de polyéthylène glycol est comprise entre 200 et 8000.

8. Comprimé à libération contrôlée de type pompe osmotique à double couche de bicyclol selon la revendication 6, où les plastifiants comprennent le polyéthylène glycol et la masse moléculaire de polyéthylène glycol est comprise entre 400 et 4000.

9. Comprimé à libération contrôlée de type pompe osmotique à double couche de bicyclol selon l'une quelconque des revendications 1 ou 6 à 8, où d'autres excipients connus de l'homme du métier pharmaceutique peuvent être ajoutés si nécessaire à la solution d'enrobage, y compris un ou plusieurs choisis dans le groupe constitué par les suivants : agents liants, lubrifiants, agents de coloration, agents opacifiants et tensioactifs.

10. Comprimé à libération contrôlée de type pompe osmotique à double couche de bicyclol selon la revendication 1, **caractérisé en ce qu'**il existe un ou plusieurs orifices de libération de médicaments sur la pellicule d'enrobage insoluble, et **en ce que** les orifices de libération de médicaments s'ouvrent à la surface de la couche de médicaments, où les orifices de libération de médicaments sont obtenus par forage laser ou autre procédé de forage mécanique.

11. Comprimé à libération contrôlée de type pompe osmotique à double couche de bicyclol selon la revendication 10, où les orifices de libération de médicaments sont obtenus par un procédé de forage laser.

12. Procédé d'élaboration du comprimé à libération contrôlée de type pompe osmotique à double couche de bicyclol selon la revendication 1, **caractérisé en ce qu'**il comprend les trois étapes suivantes : (1) préparation des comprimés à double couche, (2) enrobage par la pellicule de libération contrôlée, et (3) forage de la pellicule de libération contrôlée, où les comprimés à double couche sont élaborés par compression après granulation ou compression de poudre directe.

13. Procédé selon la revendication 12, comprenant en outre l'enrobage par un enrobage résistant à l'humidité après le forage de la pellicule de libération contrôlée.
